# EUROPEAN PATENT APPLICATION

(11) **EP 1 112 776 A1**
(43) Date of publication of application: **04.07.2001**
(21) Application number: 00403683.6
(22) Date of filing: 27.12.2000
(51) Int. Cl.: B01J 23/656, C07D 307/08

(54) **Pd-Re catalyst for hydrogenating dicarboxylic acids**

(30) Priority: 28.12.1999 JP 37436799; 15.06.2000 JP 2000180324
(71) Applicant: Tonen Chemical Corporation, Minato-Ku, Tokyo (JP)
(72) Inventor: Hamashima, Nagato, Bunkyou-Ku, Tokyo (JP); Ishihara, Takeshi, Kawagoe-Shi, Saitama-Ken (JP); Kobayashi, Kenji, Iruma-Gun, Saitama-Ken (JP); Ichiki, Tatsumi, Yokohama-Shi, Kanagawa-Ken (JP)
(74) Representative: Leszczynski, André

(57) **Abstract**

A catalyst for hydrogenating a dicarboxylic acid, a dicarboxylic acid anhydride, or an ester thereof, said catalyst comprising palladium and rhenium supported on a carrier, characterized in that not more than 20% of analyzed points have an intensity ratio of 2 or larger, wherein a catalyst particle is analyzed by electron probe microscope analysis(EPMA) for rhenium along a longest diameter in a largest cross-section of the catalyst particle, and a rhenium intensity at each analyzed point is divided by an average intensity of all of the analyzed points to give the intensity ratio, and that the carrier is at least one active carbon selected from the group consisting of coal-based active carbon, coconut-based active carbon and peat-based active carbon.

## Description

### BACKGROUND OF THE INVENTION

### FIELD OF THE INVENTION

The present invention relates to a catalyst for hydrogenating a dicarboxylic acid, a dicarboxylic acid anhydride, or an ester thereof, which may hereinafter be collectively referred to as "dicarboxylic acids." The present invention relates also to a method for hydrogenating the dicarboxylic acids using the catalyst.

### DESCRIPTION OF THE PRIOR ART

As described in International Publication WO 98/26867, it has been believed that, where a reaction rate is affected by a diffusion rate of reactants in micropores of a catalyst carrier, such a catalyst is better that a noble metal component exists as close to an outer surface of the carrier as possible, and is present less in a central part of the catalyst where the effect of diffusion prevails. Based on the aforesaid point of view, the publication discloses a catalyst carrier for a noble metal, wherein palladium is present near an outer surface of the carrier, but substantially absent inside the carrier.

National Phase Publication Hei-1-503459, Comparative Examples 17 to 19, discloses that maleic anhydride is hydrogenated using a catalyst wherein 3% of Pd and 3% of Re are supported on graphitized carbon having a large surface area. In Comparative Examples 17 and 18, selectivity to γ -butyrolactone, 1,4-butanediol and tetrahydrofuran based on reacted maleic anhydride is higher than 97%. However, the hydrogenation is performed at a pressure of 80 bar and a temperature of 230 °C, which relatively severer conditions are necessary for high yield and a high selectivity. In Comparative Example 19, the hydrogenation is performed at a pressure of 725 psi, i.e., about 50 bar, at a temperature from 200 to 260 °C. Although the pressure is lower than those in Comparative Examples 17 or 18, the selectivity to γ -butyro-lactone, 1,4-butanediol and tetrahydrofuran based on reacted maleic anhydride is so low as 43 %.

### SUMMARY OF THE INVENTION

An object of the present invention is to provide a novel palladium-rhenium catalyst for hydrogenating dicarboxylic acids, which catalyst has a very high capability of hydrogenating dicarboxylic acids and a high selectivity to lactones and to products hydrogenated further, which may hereinafter be collectively referred to as "lactones", even at a considerably low hydrogenation pressure,

As a result of intensive researches, the present inventors have found that a catalyst wherein rhenium is rather uniformly distributed throughout a carrier made of specific active carbon has a very good hydrogenating capability for dicarboxylic acids and a very high selectivity to lactones even in milder hydrogenation conditions, particularly at a lower pressure, which finding is in contradiction to the aforesaid conventional view that catalytic metals should exist as close to an outer surface of the carrier as possible rather than at a central part of the carrier.

Thus, the present invention is
(1) a catalyst for hydrogenating a dicarboxylic acid, a dicarboxylic acid anhydride, or an ester thereof, said catalyst comprising palladium and rhenium supported on a carrier, characterized in that not more than 20% of analyzed points have an intensity ratio of 2 or larger, wherein a catalyst particle is analyzed by electron probe microscope analysis (EPMA) for rhenium along a longest diameter in a largest cross-section of the catalyst particle, and a rhenium intensity at each analyzed point is divided by an average intensity of all of the analyzed points to give the intensity ratio, and that the carrier is at least one active carbon selected from the group consisting of coal-based active carbon, coconut-based active carbon and peat-based active carbon.
   The present catalyst described above has a uniform distribution of rhenium in the carrier, which distribution is not found in conventional palladium-rhenium catalysts. It has not been clarified what mechanism gives such a uniform distribution. The present inventors have found that such distribution of rhenium is realized only with at least one active carbon selected from the group consisting of coal-based active carbon, coconut-based active carbon and peat-based active carbon for the carrier.
   More surprisingly, by using the active carbon, palladium is distributed very close to an outer surface of the carrier without being distributed at a central part of the carrier while the uniform distribution of rhenium in the carrier is attained. The present catalyst has an excellent hydrogenation capability for dicarboxylic acids and a very high selectivity to lactones even in mild hydrogenation conditions, inter alia, at a very low hydrogenation pressure. These are totally unexpected from conventional palladium-rhenium catalysts, e.g., those described in the aforesaid National Phase Publication Hei-1-503459.
   The preferred embodiments of the present invention are as follows.
(2)The catalyst described in (1) above, wherein not more than 10% of all of the analyzed points have the intensity ratio of 2 or larger.
(3) The catalyst described in (1) or (2) above, wherein not more than 30% of all of the analyzed points have the intensity ratio of 0.5 or smaller.
(4) The catalyst described in (1) or (2) above, wherein not more than 20% of all of the analyzed points have the intensity ratio of 0.5 or smaller.
(5) The catalyst described in any one of (1) to (4) above, wherein a weight ratio of palladium to rhenium is in the range of from 1:0.25 to 1:10.
(6) The catalyst described in any one of (1) to (4) above, wherein a weight ratio of palladium to rhenium is in the range of from 1:0.25 to 1:5.
(7) The catalyst described in (6) above for producing γbutyrolactone.
(8) The catalyst described in any one of (1) to (4) above, wherein a weight ratio of palladium to rhenium is in the range of from 1:0.5 to 1:10.
(9) The catalyst described (8) above for producing tetrahydrofuran and/or 1,4-butanediol.
(10) The catalyst described in any one of (1) to (9) above, wherein the palladium supported is in an amount of from 0.01 to 15 wt%, based on the catalyst weight, and the rhenium supported is in an amount of from 0.1 to 20 wt%, based on the catalyst weight.
(11) The catalyst described in any one of (1) to (9) above, wherein the palladium supported in an amount of from 0,1 to 10 wt% , based on the catalyst weight, and the rhenium supported is in an amount of from 0.1 to 15 wt%, based on the catalyst weight.
(12) The catalyst described in any one of (1) to (11) above, wherein the dicarboxylic acid, the dicarboxylic acid anhydride, and the esters thereof are selected from the *group* consisting of dicarboxylic acids having 4 carbon atoms, dicarboxylic acid anhydrides having 4 carbon atoms, and esters thereof.
(13) The catalyst described in any one of (1) to (12) above, wherein the dicarboxylic acid, the dicarboxylic acid anhydride, and the esters thereof are selected from the group consisting of succinic anhydride, maleic anhydride and esters thereof.
   The present invention relates also to
(14) a method for hydrogenating a dicarboxylic acid, a dicarboxylic acid anhydride, or an ester thereof, wherein the catalyst described in any one of (1) to (13) above is used as a catalyst.
   preferred embodiments of the above invention are as follows.
(15) The method described (14) above, wherein the dicarboxylic acid, the dicarboxylic acid anhydride, and the esters thereof are selected from the group consisting of succinic anhydride, maleic anhydride and esters thereof.
(16) A method for hydrogenating a dicarboxylic acid, a dicarboxylic acid anhydride, or an ester thereof, wherein the hydrogenation is carried out at a temperature from 160 to 230 °C at a pressure from 0.5 to 9 MPa in the presence of the catalyst described in(6) above, whereby γ -butyrolactone is selectively produced.
(17) A method for hydrogenating a dicarboxylic acid, a dicarboxylic acid anhydride, or an ester thereof, wherein the hydrogenation is carried out at a temperature from 180 to 250 °C at a pressure from 0.5 to 9 MPa in the presence of the catalyst described in(8) above, whereby tetrahydrofuran is selectively produced.
(18) A method for hydrogenating a dicarboxylic acid, a dicarboxylic acid anhydride, or an ester thereof, wherein the hydrogenation is carried out at a temperature from 180 to 250 °C at a pressure from 1 to 9 MPa in the presence of the catalyst described in(8) above, whereby 1,4-butanediol is selectively produced.

### BRIEF DESCRIPTION OF THE DRAWINGS

Fig. 1 is a cumulative histogram from a ratio of rhenium intensity at each point analyzed to an average intensity, determined by EPMA on the catalyst obtained in Example 1.

Fig.2 is a cumulative histogram from a ratio of rhenium intensity at each point analyzed to an average intensity, determined by EPMA on the catalyst obtained in Example 2.

Fig.3 is a cumulative histogram from a ratio of rhenium intensity at each point analyzed to an average intensity, determined by EPMA on the catalyst obtained in Example 3.

Fig.4 is a cumulative histogram from a ratio of rhenium intensity at each point analyzed to an average intensity, determined by EPMA on the catalyst obtained in Example 4.

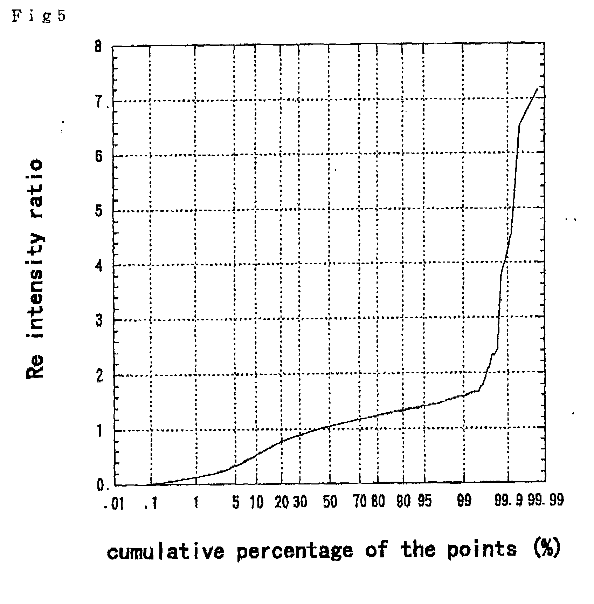

Fig.5 is a cumulative histogram from a ratio of rhenium intensity at each point analyzed to an average intensity, determined by EPMA on the catalyst obtained in Example 5.

Fig.6 is a cumulative histogram from a ratio of rhenium intensity at each point analyzed to an average intensity, determined by EPMA on the catalyst obtained in Example 6.

Fig.7 is a cumulative histogram from a ratio of rhenium intensity at each point analyzed to an average intensity, determined by EPMA on the catalyst obtained in Comparative Example 1.

### DESCRIPTION OF THE PREFERRED EMBODIMENTS

In the present catalyst comprising palladium and rhenium supported on a carrier, it is preferred that rhenium is distributed almost uniformly throughout the carrier. In the present invention, a catalyst particle is analyzed for rhenium by electron probe microscope analysis (EPMA) along a longest diameter in a largest cross-section of the catalyst particle, and a ratio of intensity at each analyzed point to an average intensity of all of the analyzed points is calculated. The ratio of the number of points having the intensity ratio of at least 2 to the total number of the analyzed points shall be at most 20 %, preferably at most 10 %, and a ratio of points having the intensity ratio of at most 0.5 to the total number of points is preferably at most 30 %, more preferably at most 20 %. If the ratio of the points having the intensity ratio of at least 2 is out of the aforesaid range, rhenium is distributed unevenly in the carrier and the catalyst has neither a high hydrogenation capability for dicarboxylic acids nor a high selectivity to γbutyrolactone and so on. In contrast to rhenium, palladium exists preferably mainly close to an outer surface of the carrier.

The analysis by EPMA is made along a longest diameter in a largest cross-section of a catalyst particle. As an EPMA instrument, EPM 8100, ex Shimazu Co., for instance, may be used. The largest cross-section and the longest diameter are determined by visual observation with an electron microscope. Particles which seem deformed under visual observation under an electron microscope are removed, and three particles are chosen at random from the remaining particles. The analysis is conducted on the selected three particles. EPMA conditions are as follows.
Acceleration voltage : 15 kV
Sampling step : 1 *µ*m
Measurement time : 1 sec/point
Sample current : 0.5 *µ*A

A characteristic X-ray chart of rhenium is obtained by EPMA analysis along the longest diameter in the largest cross-section of the catalyst particle. Signal intensity at each analyzed point(characteristic X-ray intensity) reflects the amount of supported rhenium at the point. The intensity data thus obtained are statistically processed to numerically express the supported state of rhenium. That is, the intensity at each point is divided by an average intensity of all of the analyzed points, and a cumulative histogram from the thus-obtained intensity ratios is made. The histogram shows a cumulative percentage of the points below a certain intensity ratio.

Besides the EPMA line analysis, distribution of rhenium can be analyzed by X-ray photoelectron spectroscopy (XPS), or scanning electron microscope-energy dispersive x-ray spectroscopy (SEM-EDS).

In the present catalyst, the carrier on which palladium and rhenium are supported is at least one active carbon selected from the group consisting of coal-based active carbon, coconut-based active carbon, and peat-based active carbon. Coal-based active carbon and coconut-based active carbon are preferred because they provide higher selectivity for γ -butyrolactone. The active carbon may be in a form of powder, granule, or a molded article and preferably has an average particle size of 100 *µ*m or more, more preferably from 500 *µ*m to 30 mm, for easy handling and filtering.

In the present invention, palladium is supported preferably in an amount of from 0.01 to 15 wt%, more preferably from 0.1 to 10 wt%, particularly from 0.5 to 8 wt%, based on a weight of the catalyst. Rhenium is supported preferably in an amount of from 0.01 to 20 wt%, more preferably from 0.1 to 15 wt%, particularly from 0.5 to 10 wt%, based on a weight of the catalyst. If either element is contained in an amount less than the aforesaid lower limit, hydrogenation capability is undesirably low. If either element is contained in an amount more than the aforesaid higher limit, production costs is undesirably high. A weight ratio of palladium to rhenium is preferably in the range of from 1:0.25 to 1:10. If the ratio is larger than the aforesaid higher limit, conversion of dicarboxylic acids is lower. If the ratio is smaller than the aforesaid lower limit, an excess amount of rhenium does not improve the catalyst performance, but raises costs. Preferably, the aforesaid ratio of palladium to rhenium is determined depending on a desired product. Preferably the weight ratio is in the range of from 1:0.25 to 1: to selectively produce γ -butyrolactone; from 1:0.5 to 1:10 to selectively produce tetrahydrofuran; and from 1:0.5 to 1:10 to selectively produce 1,4-butanediol.

The present catalyst may be produced, for example, by impregnating the carrier with a solution containing at least one palladium compound and at least one rhenium compound in one or multiple steps. The impregnation may be carried out by soaking or suspending the carrier in a solution of a palladium compound and a rhenium compound. Then, the solution is removed by filtration and the impregnated carrier is dried preferably at a temperature from 80 to 150°C, The solution containing the palladium compound and the rhenium compound is preferably an aqueous solution containing desired amounts of palladium and rhenium and, if necessary, other solvent such as acetonitrile; alcohols such as methanol and ethanol; ketones such as acetone; or ethers such as diethyl ether and tetrahydrofuran. Examples of the palladium compound include palladium nitrate, palladium chloride, palladium carbonate, palladium carboxylate, palladium acetate, palladium acetylacetate, and amine complex of palladium. Examples of the rhenium compound include perrhenic acid, ammonium perrhenate, and alkali metal salts of perrhenic acid. After being impregnated with palladium and rhenium and dried, the catalyst is activated by heating in a reducing atmosphere, e.g., in hydrogen gas, preferably at a temperature of from 120 to 350°C, particularly from 150 to 300 °C.

The present catalyst is advantageously used for producing lactones by hydrogenating dicarboxylic acids. Examples of the dicarboxylic acids include saturated or unsaturated dicarboxylic acids, dicarboxylic acid anhydrides, and esters thereof, preferably having 3 to 7, particularly 4, carbon atoms. Preferred are succinic acid, maleic acid, fumaric acid, succinic anhydride and maleic anhydride, among which succinic anhydride and maleic anhydride are particularly preferred.

The hydrogenation product in the present invention include lactones and further hydrogenated substances. When succinic anhydride and/or maleic anhydride are used as the dicarboxylic acid, the hydrogenation products include γ -butyrolactone as the lactone, and tetrahydrofuran and 1,4-butanediol which are hydrogenation products of γbutyrolactone.

In the present process, hydrogenation conditions maybe as follows. The hydrogenation temperature is preferably in the range of from 120 to 300 °C, particularly from 160 to 250 °C, and the pressure in the hydrogenation reaction system is preferably in the range of from atmospheric pressure to 20 MPa in gage, particularly from 0.5 to 9 MPa. Preferably, the hydrogenation conditions are selected depending on the weight ratio of palladium to rhenium and desired products.

To selectively produce γ -butyrolactone, the temperature is preferably in the range of from 160 to 230 °C, particularly from 170 to 220 °C, and the pressure is preferably in the range of from 0.5 to 9 MPa. To selectively produce tetrahydrofuran, the temperature is preferably in the range of from 180 to 250 °C and the pressure is preferably in the range of from 0.5 to 9 MPa. To selectively produce 1,4-butanediol, the temperature is preferably in the range of from 180 to 250 °C and the pressure is preferably in the range of from 1 to 9 MPa.

A molar ratio of hydrogen to the dicarboxylic acids is preferably in the range of from 3 to 5,000, particularly from 5 to 1,500, A gas space velocity is preferably in the range of from 100 to 30,000 hr⁻¹, particularly from 200 to 15,000 hr⁻¹, and a liquid space velocity is preferably in the range of from 0.05 to 10 hr⁻¹, particularly from 0.1 to 5 hr⁻¹.

In the present hydrogenation of the dicarboxylic acids, a solvent may or may not be present. By using the solvent, heat of the reaction can be easily removed, so that operations are easier. As the solvent, any solvent conventionally used in the hydrogenation of the dicarboxylic acids can be used. Examples of the solvent include γ -butyrolactone; water; ethers such as dioxane, tetrahydropyran, tetrahydrofuran, diethyl ether, and triglyme; alcohols such as methanol, ethanol, isopropanol, n-propanol, hexanol, n-butanol and isobutanol; aromatic or cycloaliphatic carboxylic acid esters such as diisobutyl ester or dibutyl ester of o-, m-, or p-phthalic acid or hexahydrogenated o-, m-, or p-phthalic acid. Preferably, γ-butyrolactone is used.

In the hydrogenation in the presence of the solvent, a concentration of the dicarboxylic acids subjected to the hydrogenation is preferably at most 90 wt %, more preferably at most 60 wt % and preferably at least 1 wt %, more preferably at least 3 wt %, particularly at least 5 wt %, based on a total weight of the dicarboxylic acids and the solvent.

The hydrogenation reaction can be performed either in a batch process or a continuous process. The present catalyst may be used in a fixed bed or a fluidized bed.

The present inventions will be further explained in the following Examples, but are not limited to those.

### EXAMPLES

### Example 1. Preparation and EPMA line analysis of 2%Pd-2.5%Re on coal-based active carbon

In a mixture of 6.72 g of acetonitrile and 21.9 g of distilled water, 3.358 g of Pd (NO₃)₂ and 2.466 g of Re₂O₇ were homogeneously dissolved, to which distilled water was added to a total volume of 64 ml. Coal-based active carbon available under the trade name, X-8000, ex Takeda Pharmaceutical Co., was ground to particles, and particle having a size of from 1 to 2 mm were selected and used as a carrier.

Seventy two grams of the carrier was impregnated with the aforesaid solution and allowed to stand at room temperature for 4 hours and then heated at 120 °C for 15 hours. The obtained catalyst precursor was heated from room temperature to 280 °C over 11 hours in a hydrogen gas flow, and kept at the temperature for 5 hours and, then, allowed to cool to room temperature. After completely purging hydrogen gas with nitrogen gas by switching the hydrogen gas flow to a nitrogen gas flow, the catalyst was placed in a nitrogen gas flow containing 5 % by volume of oxygen until heat generation ended and the catalyst became stable.

EPMA line analysis in a depth direction was made on a catalyst particle along a maximum diameter in a maximum cross-section of the particle. The particles were embedded in a polyester resin and dry polished by a rotary polishing unit to obtain a smooth cross sectional surface of the particle. Analysis conditions were as follows.
EPMA instrument ; EPM 8100, ex Shimazu Co.
Acceleration voltage : 15 kV
Sampling step: 1 *µ*m
Measurement time : 1 sec/point
Sample current : 0.5 *µ*A
Electron beam diameter : 1 *µ*m

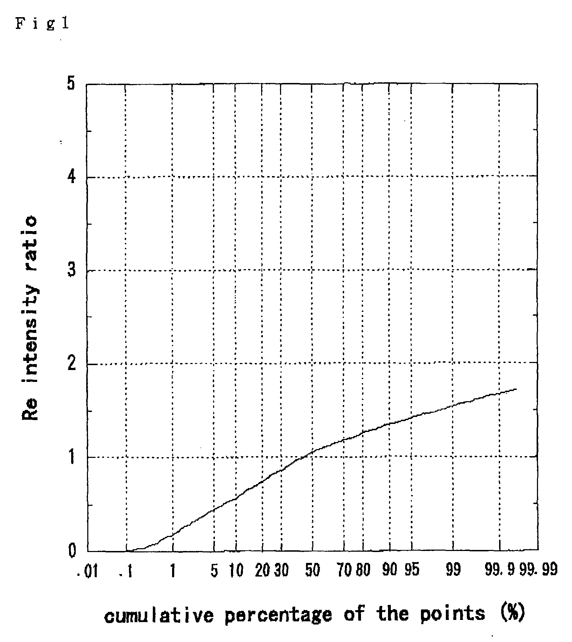

Fig.1 shows a cumulative histogram from a ratio of intensity at each analyzed point to an average intensity of all of the analyzed points. About 10 % of the points had the intensity ratio of at most 0.5, and there is no point having the intensity ratio of 2 or larger. This indicates that there are only a small number of points where a rhenium content is far less or more than the average content and , therefore, rhenium is distributed substantially uniformly in the coal-based active carbon carrier.

Semi-quantitative analysis by XPS(X-ray Photoelectron Spectroscopy) was made on an outer surface and a cross-section of the catalyst particle. Analysis conditions were as follows.
XPS instrument: Model 5600 ci, ex Physical Electronics Co.
X-ray source : conventional Al Kₒ 300 W
Diameter of analyzed area: 800 *µ*m

The XPS analysis found that rhenium existed in an amount of 0.3 atom % both at the outer surface and in the cross-section . This indicates that rhenium was distributed uniformly in the coal-based active carbon carrier.

### Example 2. Preparation and EPMA line analysis of 2%Pd-2.5%Re on coconut-based active carbon

Example 1 was repeated except that coconut-based active carbon available under a trade name of Granulate Shirasagi Gc 4/8, ex Takeda Pharmaceutical Co. , was used as a carrier and EPMA analysis was made as in Example 1.

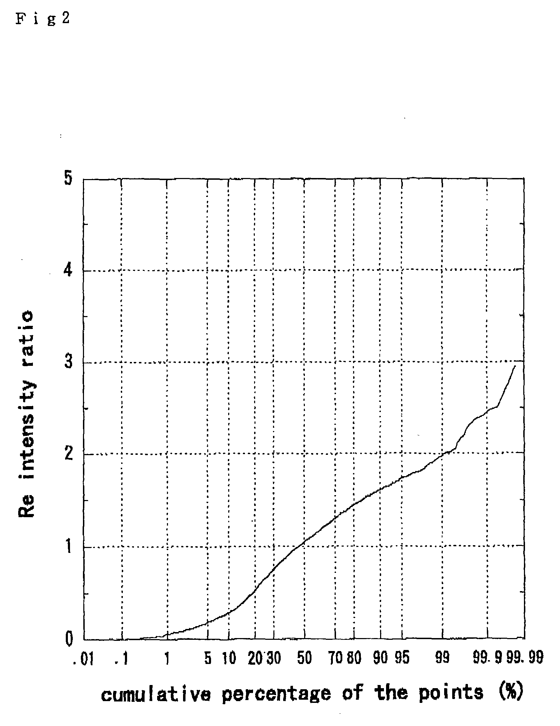

Fig.2 shows a cumulative histogram from a ratio of X-ray intensity at each point to an average intensity of all of the analyzed points. About 20 % of the points had the intensity ratio of 0.5 or smaller, and at most 1 % of the points had the intensity ratio of 2 or larger. This indicates that there are only a small number of points where a rhenium content is far less or more than the average content and , therefore, rhenium is distributed substantially uniformly in the coconut-based active carbon carrier.

Semi-quantitative analysis by XPS was conducted on a surface and the cross-section of the catalyst particle as in Example 1. The XPS analysis found that rhenium existed in an amount of 0.1 atom % at the surface and 0.3 atom % in the cross-section . This indicates that rhenium was distributed uniformly in the coal-based active carbon carrier.

### Example 3. Preparation and EPMA analysis of 2%Pd-2.5%Re on peat-based active carbon

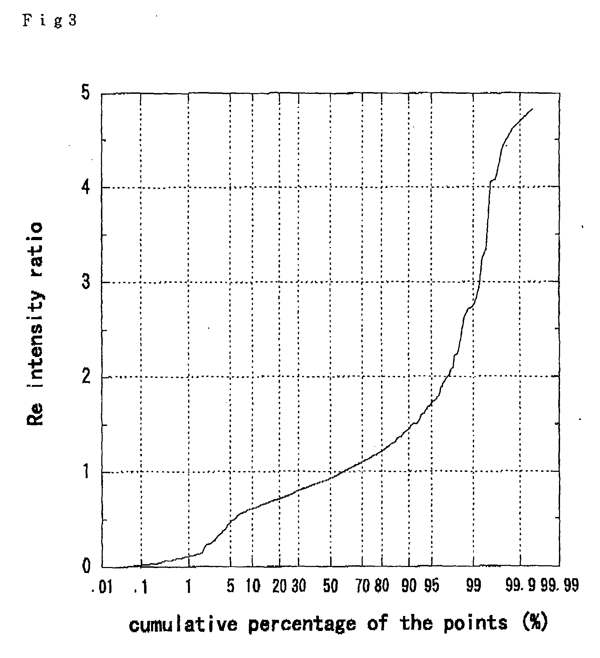

Example 1 was repeated except that peat-based active carbon available under the trade name of R.Extra, ex Nihon Norit Co., was used as a carrier and EPMA analysis was made as in Example 1. Fig.3 shows a cumulative histogram from a ratio of intensity at each point to an average intensity of all of the analyzed points. About 6 % of the points had the intensity ratio of 0.5 or smaller, and about 4 % of the points had the intensity ratio of 2 or larger. This indicates that there are only a small number of points where a rhenium content is far less or more than the average content and , therefore, rhenium is distributed substantially uniformly in the peat-based active carbon carrier.

### Example 4. Preparation and EPMA line analysis of 2%Pd-4%Re on coal-based active carbon

Example 1 was repeated except that 3.946 g of Re₂O₇ was used and EPMA analysis was made as in Example 1.

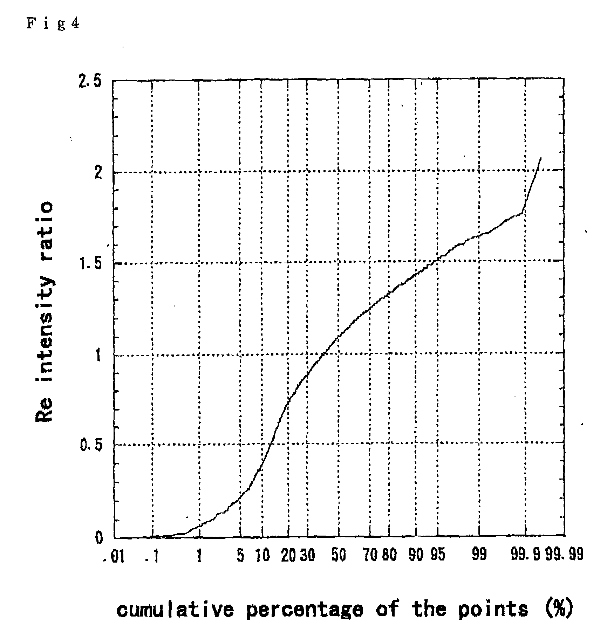

Fig.4 shows a cumulative histogram from a ratio of intensity at each point to an average intensity of all of the analyzed points. About 15 % of the points had the intensity ratio of 0.5 or smaller, and not more than 1 % of the points had the intensity ratio of 2 or larger. This indicates that there are only a small number of points where a rhenium content is far less or more than the average content and , therefore, rhenium is distributed substantially uniformly in the coal-based active carbon carrier.

### Example 5. Preparation and EPMA line analysis of 2%Pd-4%Re on coconut-based active carbon

Example 2 was repeated except that 3.946 g of Re₂O₇ was used and EPMA analysis was made as in Example 2.

Fig,5 shows a cumulative histogram from a ratio of intensity at each point to an average intensity of all of the analyzed points. About 10 % of the points had the intensity ratio of 0.5 or smaller, and not more than 1 % of the points had the intensity ratio of 2 or larger. This indicates that there are only a small number of points where a rhenium content is far less or more than the average content and , therefore, rhenium is distributed substantially uniformly in the coconut-based active carbon carrier.

### Example 6. Preparation and EPMA line analysis of 2%Pd-4%Re on peat-based active carbon

Example 3 was repeated except that 3.946 g of Re₂O₇ was used EPMA analysis was made as in Example 3.

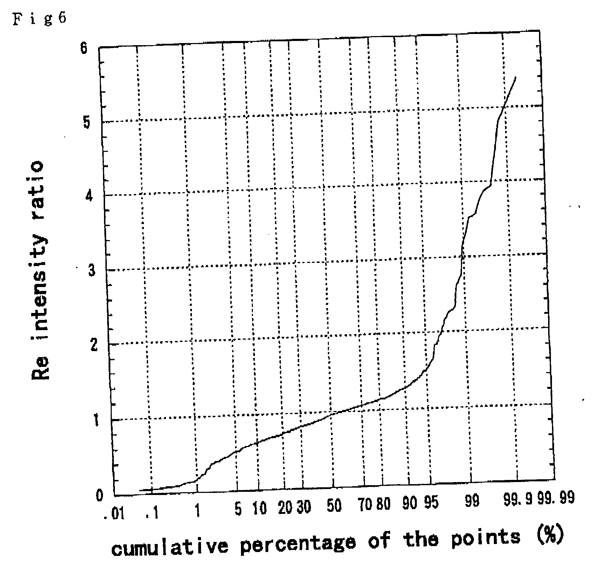

Fig.6 shows a cumulative histogram from a ratio of intensity at each point to an average intensity of all of the analyzed points. About 5 % of the points had the intensity ratio of 0.5 or smaller, and about 3% of the points had the intensity ratio of 2 or larger. This indicates that there are only a small number of points where a rhenium content is far less or more than the average content and , therefore, rhenium is distributed substantially uniformly in the peat-based active carbon carrier.

### Example 7. Preparation and EPMA line analysis of 2%Pd-6%Re on coal-based active carbon

Example 1 was repeated except that 5.919 g of Re₂O₇ was used and EPMA analysis was made as in Example 1.

From the EPMA analysis, it was found that about 13 % of the points had a ratio of intensity at each point to an average intensity of all of the analyzed points of ,0.5 or smaller and not more than 1 % of the points had the intensity ratio of 2 or larger. This indicates that there are only a small number of points where a rhenium content is far less or more than the average content and , therefore, rhenium is distributed substantially uniformly in the coal-based active carbon carrier.

### Comparative Example 1 Preparation and EPMA line analysis of 2%Pd-2.5%Re on resin-based active carbon

Example 1 was repeated except that resin-based active carbon available under the trade name of Bellfine BGF 10-1, ex Kanebo Co. ,was used and EPMA analysis was made as in Example 1.

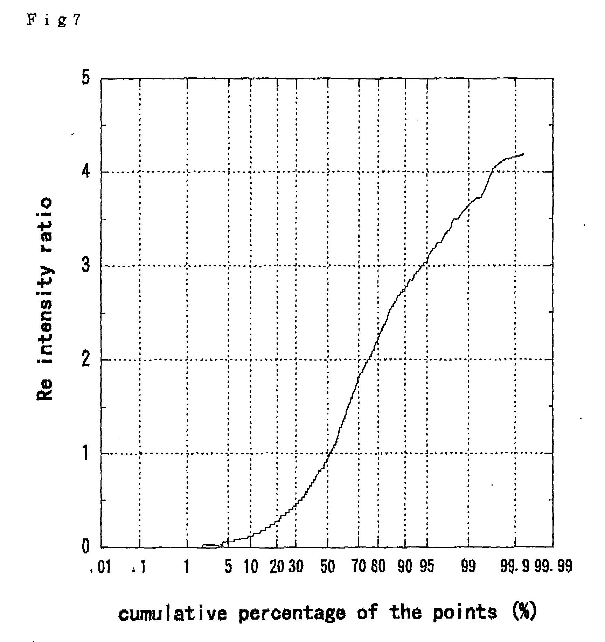

Fig.7 shows a cumulative histogram from a ratio of intensity at each point to an average intensity of all of the analyzed points. About 35 % of the points had the intensity ratio of 0.5 or smaller, and about 25% of the points had the intensity ratio of 2 or larger. This indicates that there are a relatively large number of points where a rhenium content is far less or more than the average content and , therefore, rhenium is distributed non-uniformly in the resin-based active carbon carrier.

### Examples 8 to 11 and Comparative Example 2

Using each of the catalysts prepared in Examples 1 to 3 and Comparative Example 1, hydrogenation of maleic anhydride was carried out. Each catalyst was screened and a fraction of the catalyst having a particle size of from 1 to 2mm was collected, and 10 ml of the thus collected catalyst particles were packed in a SUS 316 steel cylindrical reactor of 15 mm in inner diameter and 350 mm in length.

Then, the reactor was thoroughly purged with nitrogen gas, and a nitrogen gas flow was passed through the reactor at a flow rate of 6 liters/hour, that is, GHSV 600 h⁻¹, at an internal pressure of 0.5 MPa. Subsequently, the gas flow was changed to a flow of nitrogen gas containing 0.1 vol% of hydrogen. While letting it to pass through the reactor in the same conditions as described above, a temperature of the catalyst layer was raised to 120 °C. After confirming that the hydrogen contents at the inlet of the reactor and at the exit of the reactor became identical with each other at the temperature, the temperature of the catalyst layer was gradually raised to 160 °C. At 160 °C, a hydrogen content in the gas flow was gradually increased from 0.1 vol% to 0.5 vol%, and then the temperature was gradually raised to 170 °C. At 170 °C, the hydrogen content in the gas flow was gradually increased from 0.5 vol% to 100vol%. After the hydrogen content reached 100 %, the temperature of the catalyst layer was gradually raised to 200°C to end the reduction of the catalyst.

In the aforesaid process, the temperature of the catalyst layer and the hydrogen content in the gas flow were gradually increased so that the hydrogen contents at the inlet of the reactor and at the exit of the reactor were identical with each other. If the hydrogen contents at the inlet and at the exit of the reactor were different from each other, the temperature or the hydrogen content were kept unchanged until the hydrogen contents became identical with each other.

Next, the hydrogenation of maleic anhydride was carried out. The hydrogenation conditions were as follows.

Temperature : 180 °C in Examples 8 to 10 and Comparative Example 2, and 200 °C in Example 11
Pressure : 0.8 MPa

Hydrogen gas flow rate : 10.8 liters/hour, i.e., GHSV = 1080h⁻¹ Reaction solution : a solution prepared by dissolving 98.06 g of maleic anhydride in 258.3 g of γ -butyrolactone, i.e., 27.5 wt% MAH, was fed in the reactor at a flow rate of 2 ml/hour.

Table 1 shows the results of the hydrogenation. The whole maleic anhydride (hereinafter referred to as MAH) was immediately converted to succinic anhydride (hereinafter referred to as SAH). The subsequent step of conversion from SAH to γbutyrolactone (hereinafter referred to as GBL) and so on is a ratedetermining step, so that the conversion of SAH was used as a measure for the progress of reaction.

**Table 1**

| | | Examples | | | Comparative Examples |
|---|---|---|---|---|---|
| | 8 | 9 | 10 | 11 | 2 |
| Catalyst Pd:Re(weight ratio) | P d - R e 1:1.25 | P d - R e 1:1.25 | P d - R e 1:1.25 | P d - R e 1:1.25 | P d - R e 1:1.25 |
| Carrier | coal-based | coconutbased | peat-based | peat-based | resin-based |
| S A H conversion | 1 0 0 % | 1 0 0 % | 8 9 % | 1 0 0 % | 8 3 % |
| GBL yield | 9 6 % | 9 6 % | 8 3 % | 9 5 % | 7 4 % |
| THF¹⁾ yield | 2 % | 1 % | 0 % | 1 % | 0 % |
| other yield other yield | 2 % 2 % | 3 % 3 % | 6 % 6 % | 4 % 4 % | 9 % 9 % |

| | | | | | |
|---|---|---|---|---|---|
| ^{*}1 THF represents tetrahydrofuran. | | | | | |

In Example 8, the Pd-Re catalyst on the coal-based active carbon carrier prepared in Example 1 was used for hydrogenating MAH. The conversion of SAH, as a measure for the progress of reaction, was very high and the total yield of GBL and THF was also high. In Example 9, the Pd-Re catalyst on the coconut-based active carbon carrier prepared in Example 2 was used for hydrogenating MAH. The catalyst performance was so good, that the conversion of SAH and the total yield of GBL and THF were almost the same as those in Example 8. In Example 10, the Pd-Re catalyst on the peat-based active carbon carrier prepared in Example 3 was used for hydrogenating MAH, Although the conversion of SAH and the total yield of GBL and THF were less than those in Example 8 or Example 9, the effects of the invention were still recognized. In Example 11, Example 10 was repeated except that the temperature was 200 °C instead of 180 °C. The conversion of SAH and the total yield of GBL and THF were as high as those in Examples 8 and 9,

In comparative Example 2, the Pd-Re catalyst on the resin-based carrier prepared in Comparative Example 1 was used for hydrogenating MAH. The conversion of SAH and the total yield of GBL and THF were very low.

In the hydrogenation of MAH in Comparative Examples 17 and 18 in the aforesaid National Phase Publication Hei-1-503459, 3%Pd-3%Re catalyst supported on a graphitized carbon of a large surface area is used, and the resulting GBL yields were about 96 % and about 89 %, respectively, and a total yield of GBL, THF and 1,4-butanediol was in the range from 97 to 98%. However, the hydrogenation pressure and temperature are 80 bar or 8 MPa and 230°C, which are significantly higher than 0.8 MPa and 180°C or 200 °C in the present Examples 8 to 11. In Comparative Example 19 in National Phase Publication Hei-1-503459, the hydrogenation pressure was 725 psi or about 50 bars or about 5 MPa and the temperature was in the range from 200 to 260°C. Thus, the pressure is a little lower than those in Comparative Examples 17 and 18. However, GBL yield is as low as 36% and the total yield of GBL, THF and 1,4-butanediol is also so low as 43%.

As described above, a very high GBL yield and a very high conversion of MAH were attained by using the present catalyst. A GBL yield and a conversion of MAH comparable to those with conventional catalysts were obtained in very milder conditions, particularly at a low pressure of about 1 tenth of a conventionally employed pressure.

### Examples 12 to 18 and Comparative Example 3

Hydrogenation of MAH was carried out using each of the catalysts prepared in Examples 1 to 7 and in Comparative Example 1. Each catalyst was screened and a fraction of the catalyst having a particle size of from 1 to 2mm was collected, and 10 ml of the thus collected catalyst particles was packed in a SUS 316 steel cylindrical reactor of 15 mm in inner diameter and 600 mm in length.

Then, the reactor was thoroughly purged with nitrogen gas, and a nitrogen gas flow was passed through the reactor at a flow rate of 15 liters/hour, that is, GHSV 1500 h⁻¹, at an internal pressure of 0.3 MPa. Subsequently, the gas flow was changed to a flow of nitrogen gas containing 0.1 vol% of hydrogen. While letting it to pass through the reactor in the same conditions as described above, a temperature of the catalyst layer was raised to 120 °C. After confirming that the hydrogen contents at the inlet of the reactor and at the exit of the reactor became identical with each other at the temperature, the temperature of the catalyst layer was gradually raised to 160 °C. At 160 °C, a hydrogen content in the gas flow was gradually increased from 0.1 vol% to 0.5 vol%, and then the temperature was gradually raised to 170 °C. At 170 °C, the hydrogen content in the gas flow was gradually increased from 0.5 vol% to 100vol%. After the hydrogen content reached 100 %, the temperature of the catalyst layer was gradually raised to 200°C to end the reduction of the catalyst.

In the aforesaid process, the temperature of the catalyst layer and the hydrogen content in the gas flow were gradually increased so that the hydrogen contents at the inlet of the reactor and at the exit of the reactor were identical with each other. If the hydrogen contents at the inlet and at the exit of the reactor were different from each other, the temperature or the hydrogen content were kept unchanged until the hydrogen contents became identical with each other.

Next, the hydrogenation of maleic anhydride was carried out. The hydrogenation conditions were as follows.
Temperature : 200°C

Pressure : 8.5 MPa (Example 13), and about 7 MPa (except Example 13) Hydrogen gas flow rate : 40 liters/hour, i.e., GHSV = 4000 h⁻¹ Reaction solution : a solution prepared by dissolving 98.06 g of maleic anhydride in 258.3 g of γ -butyrolactone, i.e., 27.5 wt% MAH, was fed in the reactor at a flow rate of 4 ml/hour.

The results are as shown in Table 2. The conversion of SAH was used as a measure for the progress of reaction as in Examples 8 to 11.

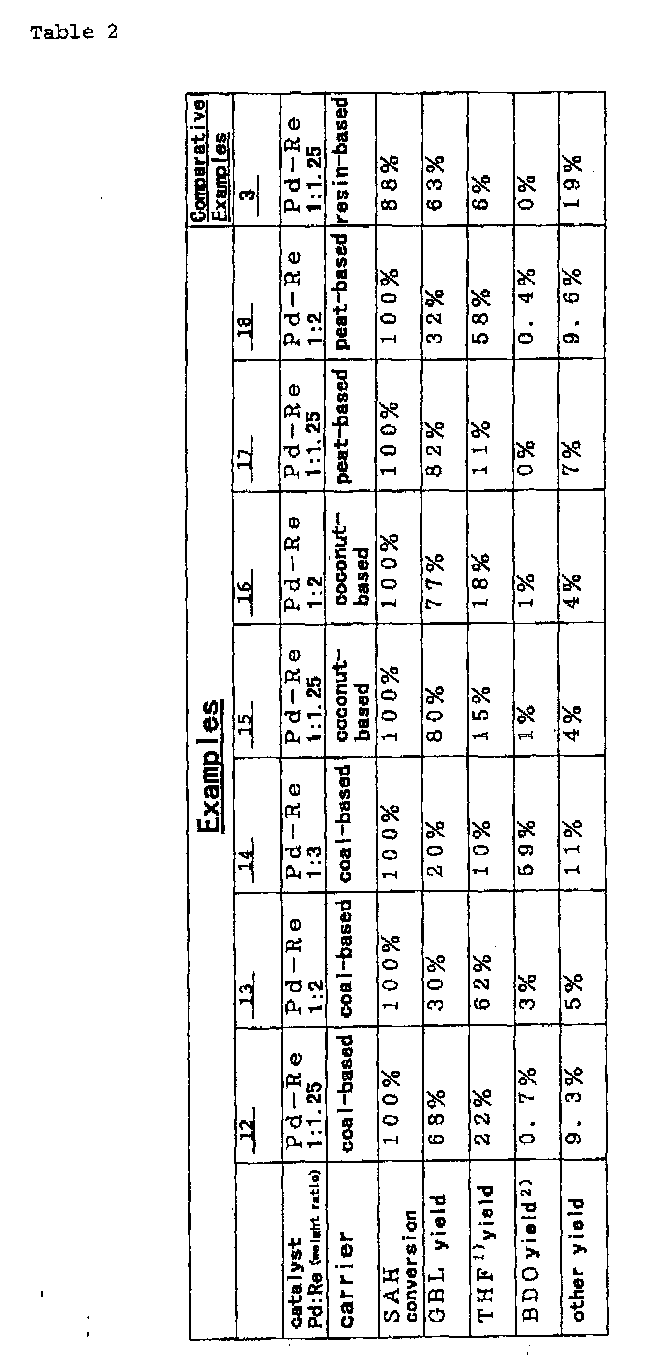

^{*}2 BOD represents 1,4-butanediol.

In Example 12, the hydrogenation of MAH was performed using the catalyst on the coal-based active carbon support having a ratio of Pd to Re of 1:1.25 prepared in Example 1. The conversion of SAH as a measure for the progress of reaction, was very high and the total yield of GBL and THF was also high. In Example 12, the hydrogen pressure was 7 MPa, which was about ten times as high as 0.8 MPa in Example 8. Compared with the hydrogenation in Example 8, the hydrogenation in Example 12 advanced further, resulting in the increased yield of THF with the decreased yield of γ -butyrolactone. It was found that a yield of THF increased with an increasing hydrogen pressure.

In Example 13, the catalyst having a ratio of Pd to Re of 1:2 prepared in Example 4 was used. It was found that the yield of γ -butyrolactone decreased further and the yield of THF increased further with the decreasing ratio of Pd to Re.

In Example 14, the catalyst having a ratio of Pd to Re of 1:3 prepared in Example 7 was used. It was found that the yield of γ -butyrolactone decreased further and the yield of THF decreased here and the yield of BOD increased in turn with the further-decreasing ratio of Pd to Re.

In Example 15, the hydrogenation of MAH was performed using the catalyst on the coconut-based active carbon support having a ratio of Pd to Re of 1:1.25 prepared in Example 2. The conversion of SAH as a measure for the progress of reaction was very high and the total yield of GBL and THF was also high. Also in Example 15, the hydrogen pressure was about ten times as high as that in Example 9 and, accordingly, the yield of γ -butyrolactone decreased and the yield of THF increased.

In Example 16, the catalyst having a ratio of Pd to Re of 1:2 prepared in 5 was used. Here again it was found that the yield of γ-butyrolactone decreased further and that of THF increased with the decreasing ratio of Pd to Re.

In Example 17, the hydrogenation of MAH was performed using the catalyst on the peat-based active carbon support having a ratio of Pd to Re of 1:1.25 prepared in Example 3. The conversion of SAH as a measure for the progress of the reaction was very high and the total yield of GBL and THE was also high. Compared with the hydrogenation in Example 10, the hydrogenation in Example 17 advanced further, resulting in the increased yield of THE and the decreased yield of γ -butyrolactone.

In Example 18, the catalyst having a ratio of Pd to Re of 1:2 prepared in Example 6 was used. Here again it was found that the yield of γ -butyrolactone decreased further and that of THF increased further with the decreasing ratio of Pd to Re.

In Comparative Example 3, the hydrogenation of MAH was performed using the Pd-Re catalyst on the resin-based active carbon support prepared in Comparative Example 1. The conversion of MAH and the total yield of GBL and THE were very low.

In the hydrogenation of MAH in Comparative Examples 17 and 18 in the aforesaid National Phase Publication Hei-1-503459, the hydrogenation pressure is 80 bar, i.e., 8 MPa, and the temperature was 230°C, which are almost the same as those in the present Examples 12 to 18. However, in Comparative Examples 17 and 18, the yields of THF are less than 0.8 % and 7.6 %, respectively, while those of GBL are about 96 % and about 89%, respectively. To increase the yield of THF in Comparative Examples 17 and 18 to the level of the present Examples 12 to 18, both the hydrogenation pressure and the temperature may have *to be* raised significantly.

As described above, MAH can be successfully converted to γbutyrolactone, THF and BDO with a high yield by using the present catalyst. Selectivity to γ -butyrolactone, THF or EDO can be varied at will by changing a ratio of Pd to Re. Further, γ -butyrolactone, THF or BDO may be obtained with a higher yield in much milder conditions, particularly at a lower pressure, than those employed in the hydrogenation with conventional catalysts.

## Claims

1. A catalyst for hydrogenating a dicarboxylic acid, a dicarboxylic acid anhydride, or an ester thereof, said catalyst comprising palladium and rhenium supported on a carrier, characterized in that not more than 20% of analyzed points have an intensity ratio of 2 or larger, wherein a catalyst particle is analyzed by electron probe microscope analysis (EPMA) for rhenium along a longest diameter in a largest cross-section of the catalyst particle, and a rhenium intensity at each analyzed point is divided by an average intensity of all of the analyzed points to give the intensity ration, and that the carrier at least one active carbon selected from the group consisting of coal-based active carbon, coconut-based active carbon and peat-based active carbon.

2. The catalyst according to claim 1, wherein not more than 10% of all of the analyzed points have the intensity ratio of 2 or larger.

3. The catalyst according to claim 1, wherein not more than 30% of all of the analyzed points have the intensity ratio of 0.5 or smaller.

4. The catalyst according to claim 1, wherein not more than 20% of all of the analyzed points have the intensity ratio of 0.5 or smaller.

5. The catalyst according to claim 1, wherein a weight ratio of palladium to rhenium is in the range of from 1:0.25 to 1:10.

6. The catalyst according to claim 1, wherein a weight ratio of palladium to rhenium is in the range of from 1:0.25 to 1:5.

7. The catalyst according to claim 6, wherein the catalyst is used for producing γ -butyrolactone.

8. The catalyst according to claim 1, wherein a weight ratio of palladium to rhenium is in the range of from 1:0.5 to 1:10.

9. The catalyst according to claim 8, wherein the catalyst is used for producing tetrahydrofuran and 1,4-butanediol.

10. The catalyst according to claim 1, wherein the palladium supported is in an amount of from 0.01 to 15 wt%, based on the catalyst weight, and the rhenium supported is in an amount of from 0.1 to 20 wt%, based on the catalyst weight.

11. The catalyst according to claim 1, wherein the palladium supported in an amount of from 0 .1 to 10 wt% , based on the catalyst weight, and the rhenium supported is in an amount of from 0.1 to 15 wt%, based on the catalyst weight.

12. The catalyst according to claim 1, wherein the dicarboxylic acid, the dicarboxylic acid anhydride, and the esters thereof are selected from the group consisting of dicarboxylic acids having 4 carbon atoms, dicarboxylic acid anhydrides having 4 carbon atoms, and esters thereof.

13. The catalyst according to claim 1, wherein the dicarboxylic acid, the dicarboxylic acid anhydride, and the esters thereof are selected from the group consisting of succinic anhydride, maleic anhydride and esters thereof.

14. A method for hydrogenating a dicarboxylic acid, a dicarboxylic acid anhydride, or an ester thereof, wherein the catalyst according to any one of claims 1 to 13 is used.

15. The method according to claim 14, wherein the dicarboxylic acid, the dicarboxylic acid anhydride, and the esters thereof are selected from the group consisting of succinic anhydride, maleic anhydride and esters thereof.

16. A method for hydrogenating a dicarboxylic acid, a dicarboxylic acid anhydride, or an ester thereof, wherein the hydrogenation
is carried out at a temperature from 160 to 230 °C at a pressure from 0.5 to 9 MPa in the presence of the catalyst according to claim 6, whereby γ -butyrolactone is selectively produced.

17. A method for hydrogenating a dicarboxylic acid, a dicarboxylic acid anhydride, or an ester thereof, wherein the hydrogenation is carried out at a temperature from 180 to 250 °C at a pressure from 0.5 to 9 MPa in the presence of the catalyst according to claim 8, whereby tetrahydrofuran is selectively produced.

18. A method for hydrogenating a dicarboxylic acid, a dicarboxylic acid anhydride, or an ester thereof, wherein the hydrogenation is carried out at a temperature from 180 to 250 °C at a pressure from 1 to 9 MPa in the presence of the catalyst according to claim 8, whereby 1,4-butanediol is selectively produced.
